# EUROPEAN PATENT APPLICATION

(11) **EP 1 447 397 A1**
(43) Date of publication of application: **18.08.2004**
(21) Application number: 03003611.5
(22) Date of filing: 17.02.2003
(51) Int. Cl.: C07C 313/02, C07C 303/02, C07C 303/36, C07C 303/28, C07C 309/81, C07C 311/03, C07C 311/14, C07C 309/67, C07D 277/52, A61K 31/18, A61K 31/185, A61K 31/255, A61P 31/04, A61P 31/10

(54) **Polyfunctional sulfonamides and sulfonates and process for preparing them**

(71) Applicant: Ecole Polytechnique Fédérale de Lausanne, 1015 Lausanne (CH)
(72) Inventor: Vogel, Pierre, 1028 Preverenges (CH); Bouchez, Laure, 1020 Renens (CH); Dubbaka, Srinivas, 1110 Morges (CH); Turks, Maris, 1022 Chavannes-renes (CH)
(74) Representative: Stolmar, Matthias

(57) **Abstract**

The present invention relates to a process for preparing sulfonyl halides comprising the steps of a) reacting an enoxymetal, allylsilane or allylstannane compound, preferably in the presence of a diene, with sulphur dioxide and b) adding a suitable halogen source to the metal sulfinate intermediate of step a). Furthermore, the present invention relates to new sulfonyl chlorides, bromides, and iodides, sulfonamides and sulfonates. In addition, the present invention teaches the use of the new multifunctional sulfonamides and sulfonates for the preparation of a medicament, bacteriocides, fungicides, pesticides, and herbicides as well as the use of sulfonyl chlorides for the preparation of sulfonamides and sulfonates.

## Description

### Field of the invention

The present invention relates to a process for preparing sulfonyl halides comprising the steps of a) reacting an enoxymetal, allylsilane or allylstannane compound, preferably in the presence of a diene, with sulphur dioxide and b) adding a suitable halogen source to the metal sulfinate intermediate of step a). Furthermore, the present invention relates to new sulfonyl chlorides, bromides, and iodides, sulfonamides and sulfonates. In addition, the present invention teaches the use of the new multifunctional sulfonamides and sulfonates for the preparation of a medicament, bacteriocides, fungicides, pesticides, and herbicides as well as the use of sulfonyl chlorides for the preparation of sulfonamides and sulfonates.

### Background of the invention

Sulfonamides have been widely known for their biological properties. For example, sulfonamides are useful as bacteriocides, antimicrobials, insecticides, herbicides, fungicides, HIV protease inhibitors, carboanhydrase inhibitors, endothelin antagonists, fibrinogen receptor antagonists, etc.

Because of their immense commercial importance the synthesis of sulfonamides is of particular interest and numerous synthetic pathways for the many different desired derivatives of sulphonamide have been explored. One of the most general and facile synthetic routes to both unsubstituted and substituted sulfonamides is the reaction of ammonia or amines with a sulfonyl halide, most often in the presence of a suitable base. Further sulfonic derivatives that may be used are sulfonate esters, anhydrides, or even sulfonyl isocyanates and sulfonyl azides.

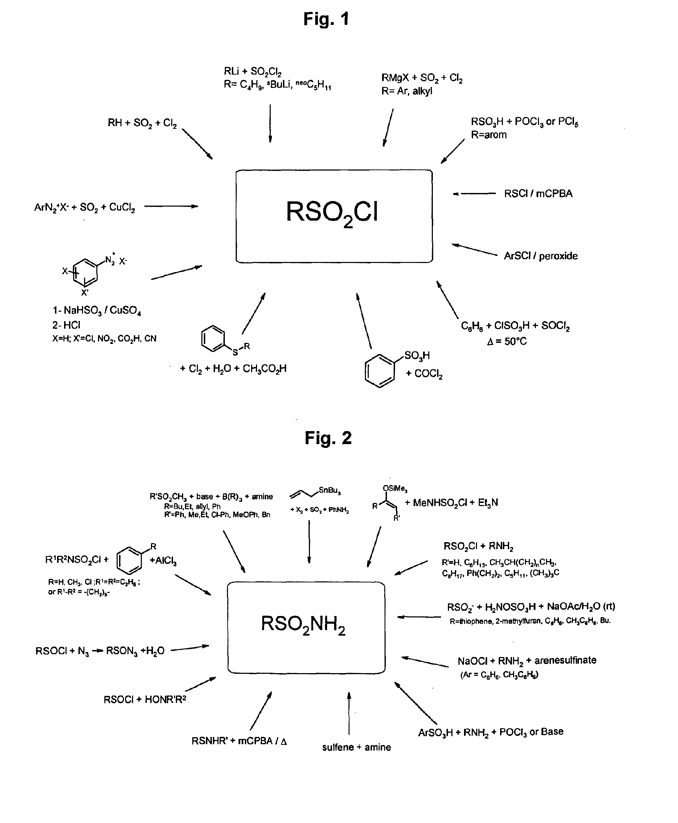

For an overview of reaction pathways that lead to sulfonamides and their most important precursors the sulfonyl halides it is referred to figures 1 (synthetic routes to sulfonyl halides) and 2 (synthetic routes to sulfonamides).

Sulfonate esters are also an important group of chemicals that elicit pharmaceutically actions.

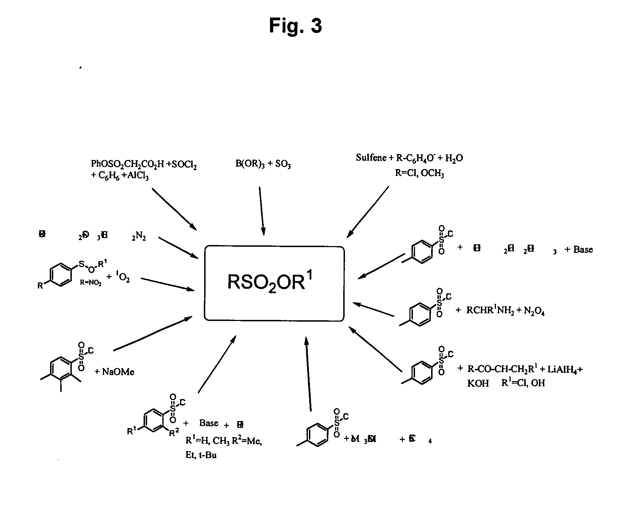

A short overview of known synthetic strategies for producing sulfonate esters is provided in Fig. 3. One of the more important synthetic routes to sulfonate esters is via the reaction of sulfonyl halides with alcohols.

Bouchez and Vogel (Synthesis 2002, No. 2, Georg Thieme Verlag Stuttgart new York, p. 225-231) discloses the reaction of silyl enol ethers of esters, ketones, as well as allylstannane and allylsilanes with sulfur dioxide that is activated by t-BuMe₂SiOSO₂CF₃ to give sulfinates that can be reacted in the same pot with a variety of electrophils generating the corresponding polyfunctional sulfones. Silyl sulfinate intermediates are formed.

Huang and Vogel (Synthesis 2002, No. 2, Georg Thieme Verlag Stuttgart new York, p. 232-236) discloses the reaction of (E,E)-1-benzyloxy-2-methylpenta-1,3-diene with trimethylsilyl enol ethers derived from acetone or acetophenone with sulfur dioxide precomplexed with ytterbium triflate or (CF₃SO₂)₂NH that generates sulfinate intermediates that can be quenched by a variety of electrophils to afford the corresponding polyfunctional sulfones in one-pot operations.

Presently, the harsh reaction conditions (e.g. highly reactive starting materials, high temperature) associated with the introduction of the sulfuryl groups into the sulfonyl halides used to prepare sulfonamides and sulfonate esters provide clear limits to structural diversity. Therefore, the number of reaction schemes available for producing polyfunctional sulfonamides and sulfonate esters is very limited and yields are often low. Furthermore, the preparation of sulfonamides and sulfonate esters most often requires multistep synthesis with intermediate purification steps.

In summary, there is a need for polyfunctional sulfonyl halides as intermediates for the preparation of sulfonamides and sulfonate esters. Furthermore, there is a need for a process for the preparation of polyfunctional sulfonyl halides that employs moderate reaction conditions. In addition, it is a further object of the invention to provide a process for the preparation of sulfonamides and sulfonate esters that is applicable to a "one pot" - synthesis strategy, wherein the sulfonyl halide moiety can be prepared in the same reaction vesicle that is afterwards employed to prepare the sulfonamide or sulfonate ester products without any intermediate purification steps. Lastly, it is an object of the invention to provide new polyfunctional sulfonamides and sulfonate esters that are useful for pharmaceutical and agricultural applications.

### Disclosure of the invention

In one aspect the present invention provides a process for preparing sulfonyl halides comprising the following steps:
a) reacting an enoxymetal, allylsilane or allylstannane compound with sulphur dioxide in the presence of a suitable Lewis acid, in a suitable solvent, at a suitable temperature, and for a time sufficient to produce a metal sulfinate,
b) adding a suitable halogen source to the metal sulfinate of step a) and reacting said components in a suitable solvent, at a suitable temperature, and for a time sufficient to produce a sulfonyl halide.

In a preferred embodiment, in step a) an enoxymetal, allylsilane or allylstannane compound, and a compound of the general formula A: 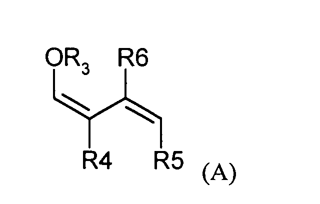 wherein
- R₃: denotes C₁₋₁₀ alkyl, aryl, arylalkyl, or hetarylalkyl, preferably C₁₋₆ alkyl, benzyl, arylmethyl, or hetarylmethyl, more preferably C₁₋₄ alkyl, H, benzyl, or furfuryl, with the proviso that any R₃ containing a free amine base or a thiol or a thiolether cannot be used.
- R₄: denotes H or C₁₋₁₀ alkyl, preferably H or C₁₋₆ alkyl, more preferably H or C₁₋₄ alkyl,
- R₅: denotes H or C₁₋₁₀ alkyl, preferably H or C₁₋₆ alkyl more preferably H or C₁₋₄ alkyl,
with the proviso that any R₄, R₅ containing a free amine, amide, thioether cannot be used.
- R₆: denotes H or acyloxy, preferably H, methylcarboxy, ethylcarboxy, ipropylcarboxy, t-butylcarboxy, or arylcarboxy, more preferably phenylcarboxy
are reacted with sulphur dioxide in the presence of a suitable Lewis acid, in a suitable solvent, at a suitable temperature, and for a time sufficient to produce a metal sulfinate.

### Step a)

The Lewis acid necessary for step a) may be any Lewis acid that will catalyze the formation of the sulfinate product. The person skilled in the art can readily select a suitable Lewis acid without undue experimentation.

In a preferred embodiment, a suitable Lewis acid for preparing sulfinates according to step a) is selected from the group consisting of BuMe₂SiOSO₂CF₃, ytterbium triflate, and (CF₃SO₂)₂NH, (CF₃SO₂)₂NSiMe₃, MgBr₂, t-Me₃SiOSO₂CF₃.

In a further preferred embodiement the enoxymetal compound is selected from Si and Sn, preferably Si.

Preferred reaction times for step a) are 30 minutes to 15 hours, more preferably 1 to 12 hours, most preferably 2 to 8 hours.

Preferred temperatures for step a) are 0 to -100°C, more preferably -20 to -100 °C, most preferably -60 to -80°C for the oxyallylation (diene + enoxysilane, allylsilane, or allylstannane + SO₂ + Lewis acid) and +49 to -100°C for the ene reaction (enoxysilane, allylsilane, or allylstannane + SO₂ + Lewis acid).

Preferred solvents for step a) are selected from the group consisting of acetonitrile and dichloromethane.

Once the sulfinate product of step a) is prepared it is preferred to evaporate the solvent and excess sulfur dioxide. No further purification is necessary. In the absence of water, corresponding silyl sulfinates can be isolated.

### Step b)

In a preferred embodiement of the invention the source of halogen in step b) is elemental halogen or a halo-succinimide compound, preferably chlorine, bromine, N-chlorosuccinimide, N-iodosuccinimide, or N-bromosuccinimide, more preferably N-chlorosuccinimide, N-iodosuccinimide, or N-bromosuccinimide, most preferably N-chlorosuccinimide.

Preferably, the temperature in step b) is 40 to -50 °C, more preferably +10 to -40 °C or -10 to -30 °C, and most preferably about -20 °C.

A suitable solvent for step b) may be any solvent that solves the reaction components without interfering with the reaction itself and one that is a fluid at the desired reaction temperature.

In a preferred embodiment the solvent in step b) is selected from the group consisting of methylene dichloride, acetonitrile, or anhydrous tetrahydrofurane, preferably methylene dichloride. And most preferably no solvent except for the source of halogen is used.

In a further preferred embodiment the reaction time in step b) is from 15 minutes to 20 hours, preferably from 30 minutes to 15 hours, more preferably from 1 hour to 8 hours, most preferably from 2 to 4 hours.

In a more preferred embodiment the process of step b) is a process according to the invention wherein no solvent is used, and the suitable temperature is -10 to -30 °C, preferably about -20 °C, and the reaction time is from 1 to 12 hours.

In a most preferred embodiment the process according to the invention is a process, wherein the product of step b) is the sulfonyl halide of
a) general formula I: 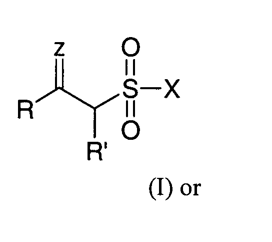
b) general formula II: 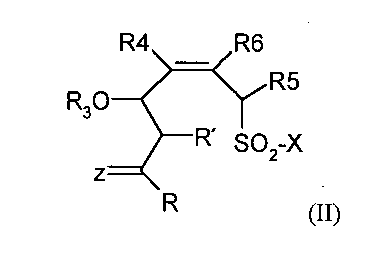
wherein
- Z: denotes CH₂ or O, preferably O
- R, R',: denote, in each case independently of one another, hydrogen, C₁₋₁₀ alkyl, C₁₋₁₀ alkoxy, aryl, hetaryl, preferably pyridine, pyrazine, pyrimidine, arylalkyl, hetarylalkyl, phenyl, or R and R" denote together a (CH₂)₃₋₆ ring or a camphoryl that may substituted by aryl, acylalkyl, hetaryl, hetarylalkyl, C₁₋₁₀ alkyl, and/or C₁₋₁₀ alkoxy, and
- R₃: denotes C₁₋₁₀ alkyl, aryl, arylalkyl, hetaryl, or hetarylalkyl, preferably C₁₋₆ alkyl, benzyl, arylmethyl, or hetarylmethyl, more preferably C₁₋₄ alkyl, H, benzyl or furfuryl, with the proviso that any R₃ containing a free amine bse or a thiol or a thiolether cannot be used.
- R₄: denotes H or C₁₋₁₀ alkyl, preferably H or C₁₋₆ alkyl, more preferably H or C₁₋₄ alkyl.
- R₅: denotes H or C₁₋₁₀ alkyl, preferably H or C₁₋₆ alkyl more preferably H or C₁₋₄ alkyl,
with the proviso that any R₄, R₅ containing a free amine, amide, thioether cannot be used.
- R₆: denotes H or acyloxy, preferably H, methylcarboxy, ethylcarboxy, i-propylcarboxy, t-butylcarboxy, or arylcarboxy, more preferably phenylcarboxy
- X: denotes halogen, preferably chlorine, bromine or iodine.

The above mentioned process according to the invention allows for the preparation of new polyfunctional sulfonyl halides under relatively mild conditions that may used directly to prepare sulfonamides and sulfonate esters by the addition of ammonia/amines or alcohols, respectively.

Therefore, in a preferred embodiment the above mentioned process according to the invention is followed by a further step c), said step c) comprising the addition of an amine to the product of step b) and reacting said components in a suitable basic solvent, at a suitable temperature, and for a reaction time sufficient to produce a sulphonamide.

Preferably, the amine is a protected amino acid, more preferably an amino acid t-butylester or the amine that has the general formula NR₁R₂ (III), wherein R₁, R₂ denote, in each case independently of one another, hydrogen, C₁₋₁₀ alkyl, aryl, arylalkyl, hetaryl, hetarylalkyl, or R₁ and R₂ form a cyclic system, preferably a saturated 3-10 or unsaturated 5-10 membered ring, more preferably benzyl.

There are a variety of suitable solvents available to the skilled person for performing step c). Preferably, the suitable solvent is pyridine or dichloromethane and a suitable base, preferably triethylamine or diethylisopropylamine.

In a preferred embodiment, ammonia or amines for reaction step c) are reacted at temperatures between 0 to -50 °C, preferably -10 to -40 °C, more preferably -10 to -30 °C, most preferably about -20 °C.

Preferably, the reaction time for the sulfonyl halide and the ammonia or amine is 30 minutes to 6 hours, preferably 1 hour to 4 hours, more preferably about 2 hours.

Alternatively, alcohols can be added to the sulfonyl halide intermediates.

In a further most preferred embodiment the above mentioned process according to invention is followed by a further step c), said step c) comprising the addition of an alcohol to the product of step b) and reacting said components in a suitable basic solvent, at a suitable temperature, and for a reaction time sufficient to produce a sulphonate ester.

While many standard solvents with or without basic ingredients are suitable for the reaction of an alcohol with the sulfonyl halide produced in step b), dichloromethane in combination with a suitable base, preferably triethylamine, NaOH or a suitable carbonate.

Preferably, the alcohol is reacted at temperatures of 0 to -50 °C, preferably -10 to -40 °C, more preferably -10 to -30 °C, most preferably about -20 °C.

Preferred reaction times for reacting the alcohol are between 30 minutes to 6 hours, preferably 1 hour to 4 hours, more preferably about 2 hours.

In a preferred embodiment the alcohol is a C₁₋₁₀ alcohol, arylalcohol, arylmethanol, hetarylmethanol, or phenol, preferably a C₁₋₆ alcohol or phenol or benzyl alcohol, more preferably a C₁₋₄ alcohol.

All of the above mentioned process steps are suitable to be employed in a so called "one pot"-reaction scheme. A "one pot"-reaction according to this invention is understood to relate to a multi-component multi-reaction snythesis in the same reaction vesicle. In particular, a "one pot"-reaction according to this invention relates to the synthesis of sulfinates according to step a) and sulfonyl halides according to step b) and optionally the preparation of sulfonamides or sulfonate esters in a further step c) in one reaction vessel without any substantial intermediate purification steps. A substantial intermediate purification step according to this invention refers to a purification process that requires the retrieval of an intermediate product from the reaction vessel.

Therefore, in a preferred embodiment all steps of a process of the present invention are performed in one reaction vessel consecutively ("one pot reaction") without substantial intermediate purification.

In a further aspect the present invention relates to a sulfonyl compound of the
a) general formula IV: 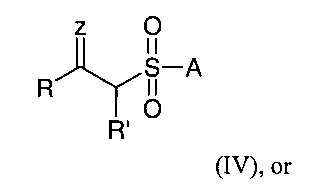
b) general formula V: 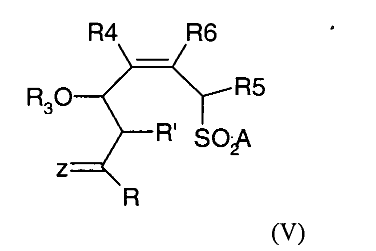
wherein
- Z: denotes CH₂ or O, preferably O
- R, R',: denote, in each case independently of one another, hydrogen, C₁₋₁₀ alkyl, C₁₋₁₀ alkoxy, aryl, hetaryl, preferably pyridine, pyrazine or pyrimidine, arylalkyl, hetarylalkyl, phenyl, or R and R" denote together a (CH₂)₃₋₆ ring or a camphoryl that may substituted by aryl, acylalkyl, hetaryl, hetarylalkyl, C₁₋₁₀ alkyl, and/or C₁₋₁₀ alkoxy, and
- R₃: denotes C₁₋₁₀ alkyl, aryl, arylalkyl, hetaryl, or hetarylalkyl, preferably C₁₋₆ alkyl, benzyl, arylmethyl, or hetarylmethyl, more preferably C₁₋₄ alkyl, H, benzyl or furfuryl with the proviso that any R₃ containing a free amine base or a thiol or a thiolether cannot be used.
- R₄: denotes H or C₁₋₁₀ alkyl, preferably H or C₁₋₆ alkyl, more preferably H or C₁₋₄ alkyl.
- R₅: denotes H or C₁₋₁₀ alkyl, preferably H or C₁₋₆ alkyl more preferably H or C₁₋₄ alkyl,
with the proviso that any R₄, R₅ containing a free amine, amide, thioether cannot be used.
- R₆: denotes H or acyloxy, preferably H, methylcarboxy, ethylcarboxy, i-propylcarboxy, t-butylcarboxy, or arylcarboxy, more preferably phenylcarboxy
- A: denotes
i) halogen, preferably chlorine, iodine or bromine, or
ii) a C₁₋₁₀ alcohol, arylalcohol, arylmethanol, hetarylmethanol, or phenol, preferably a C₁₋₆ alcohol or phenol or benzyl alcohol, more preferably a C₁₋₄ alcohol,
iii) an amino acid or an amine that has the general formula NR₁R₂ (III), wherein R₁, R₂ denote, in each case independently of one another, hydrogen, C₁₋₁₀ alkyl, aryl, arylalkyl, hetaryl, hetarylalkyl, or R₁ and R₂ form a cyclic system, preferably a saturated 3-10 or unsaturated 5-10 membered ring, more preferably benzyl.
iv) a heterocyclic system selected from the group consisting of: 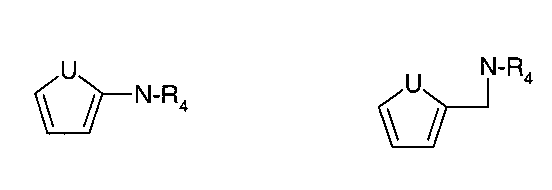 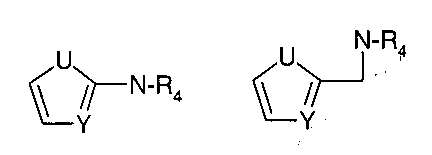 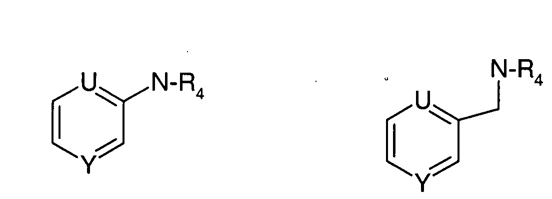
wherein
U denotes O, N-R₄, S, CH, N
Y denotes N, CH with the proviso that in a heteroyclic ring containing U and Y, U denotes N and Y denotes N or CH.

In a preferred embodiment the sulfonyl compound is characterized in that it has the general formula VI:

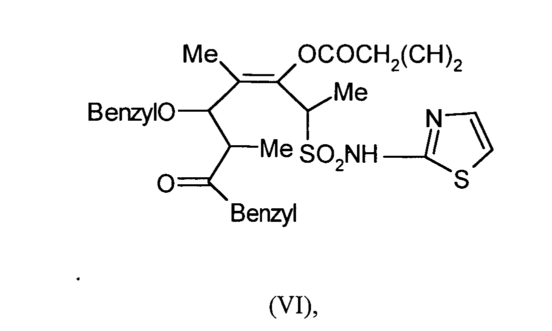

More preferably, the sulfonyl compound is : (1Z, 3R,4R)-3-(benzyloxy)-2,4 dimethyl -5-oxo-1-{1-[((1,3-thiazol-2-ylamino)sulfonyl]ethyl}hex-1-enyl carbonate.

### Sulfonate esters

In a more preferred embodiment the present invention relates to those above mentioned compounds wherein A denotes an alcohol, the compound thus being a sulfonate ester.

Most preferred compounds are selected from the group consisting of:
methyl 2-oxo-2-phenylethane sulfonate,
ethyl 2-oxo-2-phenylethane sulfonate
phenyl 2-oxo-2-phenylethane sulfonate, and
isopropyl 2-oxo-2-phenyl ethane sulfonate.

### Medical uses

In a further aspect the present invention relates to the above mentioned sulfonate ester compounds, most preferably phenyl 2-oxo-2-phenylethane sulfonate, for the preparation of a medicament.

Use of a sulfonate ester compound according to the invention for the preparation of a medicament for the treatment of bacterial or fungal infections in mammals, most preferably in humans.

A further aspect of the present invention relates to pharmaceutical composition comprising a sulfonate ester compound according to the invention.

### Sulfonamides

In a more preferred embodiment the present invention relates to those above mentioned compounds wherein A denotes an alcohol, the compound thus being a sulfonamide.

Most preferred compounds are selected from the group consisting of

Tert-butyl N-[(1,7,7-trimethyl-2-oxobicyclo [2.2.1] hept-3-yl) sulfonyl] alaninate, N,N-diethyl-1,7,7-trimethyl-2-oxo bicyclo [2.2.1] heptane-3- sulfonamide, N-benzyl-N,1,7,7-tetramethyl-2-oxo bicyclo [2.2.1] heptane-3- sulfonamide, N-benzyl-1,7,7-trimethyl-2-oxo bicyclo [2.2.1] heptane-3- sulfonamide, Tert-butyl N-[(2-oxo-2-phenyl ethyl) sulfonyl] alaninate, N-benzyl 2-oxo-2-phenyl ethane sulfonamide, N,N-diethyl 2-oxo-2-phenyl ethane sulfonamide, N, N-diethyl-3-oxopentane-2-sulfonamide, Tert-butyl N-[(2-methoxy-1-methyl-2-oxoethyl) sulfonyl] alaninate, Methyl 2- [(benzylamino) sulfonyl] propanoate, Methyl 2-{[benzyl (methyl) amino]sulfonyl)} propanoate, and Methyl 2-[(diethylamino) sulfonyl] propanoate

In a further aspect the present invention relates to the above mentioned sulfonamide compounds for the preparation of a medicament.

Use of a sulfonamide compound according to the invention for the preparation of a medicament for the treatment of bacterial or fungal infections in mammals, most preferably in humans.

A further aspect of the present invention relates to a pharmaceutical composition comprising a sulfonamide compound according to the invention.

Another aspects relates to sulfonate ester and sulfonamide compounds according to the invention that are also useful for the preparation of a bacteriocide, a pesticide, a herbicide, or a fungicide.

As demonstrated above the polyfunctional sulfonyl halides are particularly useful for the the preparation of a sulphonamide or a sulfonate, preferably under mild conditions and more preferably in a "one pot" synthesis.

Therefore, in a further aspect, the present invention relates to the use of a sulfonyl halides according to the invention for the preparation of a sulphonamide or a sulfonate.

It should be noted that the compounds useful for preparing the compounds of the present invention, the intermediates as well as the final product compounds, especially the compounds of formula (A), I, II, III, IV, and V are by no means limited to specific isomers and racemic mixtures thereof may also be employed and produced in the course of practicing the present invention.

### Figures

**Fig. 1** shows an overview of presently available synthetic pathways that lead to sulfonyl halides.
**Fig. 2** shows an overview of presently available synthetic pathways that lead to sulfonamides.
**Fig. 3** shows an overview of presently available synthetic pathways that lead to sulfonate esters.

The following examples further illustrate the best mode contemplated by the inventors for carrying out their invention. The examples relate to preferred embodiments and are not to be construed to be limiting on the scope of the invention.

### Examples

### Chemicals

All solvents were distilled prior to use: THF and Et₂O from Na and benzophenone; DMF, CH₂Cl₂, and toluene from P₂O₅. Solns after reactions and extractions were evaporated in a rotatory evaporator under reduced pressure. Liquid/solid flash chromatography (FC): columns of silica gel (0.040-0.63 µm, *Merck* No.*9385* silica gel *60*, 240-400 mesh). Thin layer chromatography (TLC) for reaction monitoring: *Merck* silica gel 60F₂₅₄ plates ; detection by UV light. *Pancaldi* reagent ((NH₄)₆MoO₄, Ce(SO₄)₂, H₂SO₄, H₂O), or KMnO₄. Reagents were from *Fluka* or *Aldrich* and used without purification. M.p.: uncorrected; *Tottoli (Büchi SMP-20*) apparatus. IR Spectra *: Perkin-Elmer-1420* or *Beckman -IR4230* spectrometer;_ in cm⁻¹. ¹H-NMR Spectra: *Bruker-DPX-400,* or *Bruker-ARX-400* spectrometer; δ(H) in ppm rel.to internal Me₄Si (= 0.00 ppm) or to the solvent's residual ¹H-signal (CH-Cl₃, δ(H) 7.27; C₆HD₅, δ(H) 7.16; CHD₂COCD₃, δ(H) 1.95; CD₂HCN, δ(H) 2.50; CHD₂SOCD₃, δ(H) 2.50, CH₂OD, δ(H) 3.31) as internal reference, all ¹H-signal assignments were confirmed by double irradiation experiments or by 2D COSY-DQF or COSY-45 spectra. ¹³C-NMR Spectra: same instruments as above (101.61MHz); δ(C) in ppm rel. to internal Me₄Si (= 0.00ppm) or to solvent's C-signal (CDCl₃, δ(C) 77.0; C₆D₆, δ(C) 128.4; (CD₃)₂CO, δ(C) 29.8; CD₃CN, δ(C) 1.3; (CD₃)₂SO, δ(C) 39.5; CD₃OD, δ(C) 49.2) as internal reference; coupling constants *J* in Hz (± 0.5 Hz). MS: *Nermag R-10-10C,* chemical ionization (NH₃) mode m/z (amu) (% rel.base peak (100%)).
Elemental analyses *: Ilse Beetz,* D-96301 Kronach, Germany.

Five different convenient procedures were developed depending on the reactivity of the starting materials.

### Preparation of sulfonylamides from sulfuryl halides

### Procedure A

This procedure was applicable to silyl enol ethers derived from esters, excepted valerolactone. (e.g. compounds 1 to 4)

(*t*-Bu)Me₂SiOSO₂CF₃ (0.14 mmol, 0.05equiv) in anh. CH₂Cl₂ (2 mL) was degassed by freeze-thaw cycles on the vacuum line. Sulfur dioxide (1.2 mL, 27.4 mmol, 10 equiv), dried by passing through column packed with phosphorus pentoxide and aluminum oxide, was transferred on the vacuum line to the CH₂Cl₂ solution frozen at -196°C. The mixture was allowed to melt and to warm to -78°C. After 30 min at this temperature the enoxysilanes (**1a**) (2.74 mmol, 1 equiv) in CH₂Cl₂ (2 mL) were added slowly. The mixture was stirred at -78°C 2 h. After cooling to -78°C, the excess of SO₂ and the solvent were evaporated under reduced pressure (10⁻³ Torr) to dryness (ca. 1h). Halogenating agent (Br₂ or NCS or NBS, 3.01 mmol, 1.1 equiv) was added at -20°C. After 1h at this temperature, the mixture was transferred to a solution of the amine (3.29 mmol, 1.2 equiv) in 3 mL pyridine or in 2 mL dichloromethane in presence of triethylamine (3.29 mmol, 1.2 equiv) under argon atmosphere. The mixture was finally stirred at this temperature for 2 h, and poured into ice-water (20 mL) and extracted with CH₂Cl₂ (15 mL, 3 times).When pyridine was used 20 mL of ether were added and the mixture was washed with a saturated solution of CuSO₄ (30 mL, 3 times) to complex the pyridine. The combined organic extracts were washed with brine (20 mL), dried (Na₂SO₄) and the solvent eliminated under reduced pressure with reflux. The residue was purified by FC.

### Procedure B

This procedure was applicable to silyl enol ethers derived from cyclic ketones.
(e.g. compound 6)

(*t*-Bu)Me₂SiOSO₂CF₃ (0.14 mmol, 0.05equiv) in anh. CH₃CN (2 mL) was degassed by freeze-thaw cycles on the vacuum line. Sulfur dioxide (1.8 mL, 41.1 mmol, 15 equiv), dried by passing through column packed with phosphorus pentoxide and aluminium oxide, was transferred on the vacuum line to the CH₃CN solution frozen at -196°C. The mixture was allowed to melt and to warm to -78°C. After 30 min at this temperature the enoxysilanes (**1d**) (2.74 mmol, 1 equiv) in CH₃CN (2 mL) were added slowly. The mixture was stirred at -78°C 3 h. After cooling to -78°C, the excess of SO₂ and the solvent were evaporated under reduced pressure (10⁻³ Torr) to dryness (ca. 1h). Halogenating agent (only NCS or NBS, 3.01 mmol, 1.1 equiv) was added at -20°C. After 1h at this temperature, the mixture was transferred to a solution of the amine (3.29 mmol, 1.2 equiv) in 3 mL pyridine under argon atmosphere. The mixture was finally stirred at this temperature for 2 h, and poured into a mixture of ice-water (10 mL) and ether (20 mL) and then washed with a saturated solution CuSO₄ (30 mL, 3 times). The combined organic extracts were washed with brine (20 mL), dried (Na₂SO₄) and the solvent eliminated under reduced pressure with reflux. The residue was purified by FC.

### Procedure C

This procedure was applicable to silyl enol ethers derived only from acyclic ketones.
(e.g. compounds 5, 7 to 14).

(*t*-Bu)Me₂SiOSO₂CF₃ (0.14 mmol, 0.05equiv) in anh. CH₃CN (2 mL) was degassed by freeze-thaw cycles on the vacuum line. Sulfur dioxide (1.8 mL, 41.1 mmol, 15 equiv), dried through column packed with phosphorus pentoxide and aluminum oxide, was transferred on the vacuum line to the CH₃CN solution frozen at -196°C. The mixture was allowed to melt and to warm to -78°C. After 30 min at this temperature the enoxysilanes (2.74 mmol, 1 equiv) in CH₃CN (2 mL) were added slowly. The mixture was stirred at -78°C 3 h. After cooling to -78°C, the excess of SO₂ and the solvent were evaporated under reduced pressure (10⁻³ Torr) to dryness (ca. 1h). Halogenating agent (Br₂ or NCS or NBS, 3.01 mmol, 1.1 equiv) was added at -20°C. After 1h at this temperature, the mixture was transferred into a solution of the amine (3.29 mmol, 1.2 equiv) in 3 mL pyridine or in 2 mL dichloromethane in presence of triethylamine (3.29 mmol, 1.2 equiv) under Argon atmosphere. The mixture was finally stirred at this temperature for 2 h, and poured into ice-water (20 mL) and extracted with CH₂Cl₂ (15 mL, 3 times).When pyridine was used 20 mL of ether were added and the mixture was washed with a saturated solution of CuSO₄ (30 mL, 3 times) to complex the pyridine. The combined organic extracts were washed with brine (20 mL), dried (Na₂SO₄) and the solvent eliminated under reduced pressure with reflux. The residue was purified by FC.

### Procedure D

This procedure was applicable to (Z)-6-oxo-4-oxyalk-2-enesulfinates.
(e.g. compound 20)

(*t*-Bu)Me₂SiOSO₂CF₃ (0.29 mmol, 0.3 equiv) in anh. CH₂Cl₂ (2 mL) was degassed by freeze-thaw cycles on the vacuum line. Sulfur dioxide (0.8 mL, 19.4 mmol, 20 equiv), dried by passing through column packed with phosphorus pentoxide and aluminum oxide, was transferred on the vacuum line to the CH₂Cl₂ solution frozen at -196°C. The mixture was allowed to melt and to warm to -78°C. After 30 min at this temperature a solution of diene (0.96 mmol, 1 equiv) and the enoxysilanes (1.26 mmol, 1.3 equiv) in CH₂Cl₂ (0.5 mL) were added dropwise under vigorous stirring and argon atmosphere. The mixture was stirred at -78°C 12 h. After cooling to -78°C, the excess of SO₂ and the solvent were evaporated under reduced pressure (10⁻³ Torr) to dryness (ca. 1h). Halogenating agent (only NCS or NBS, 3.01 mmol, 1.1 equiv) was added at -20°C. After 1h at this temperature, the dark mixture was transferred into a solution of the amine (3.29 mmol, 1.2 equiv) in 3 mL pyridine under argon atmosphere. The mixture was finally stirred at this temperature for 2 h, and poured into a mixture of ice-water (10 mL) and ether (20 mL) and then washed with a saturated solution CuSO₄ (30 mL, 3 times). The combined organic extracts were washed with brine (20 mL), dried (Na₂SO₄) and the solvent eliminated under reduced pressure with reflux. The residue was purified by FC.

**Table 1 summarizes the structural features, reaction conditions and the respective yield for compounds prepared according to the above mentioned synthesis schemes.**

**Table 1**

| **Compound** | **solvent** | **Reaction time** | **R** | **R'** | **R**^{**2**} | **R**^{**3**} | **No. of molecule** | **Yield %** |
|---|---|---|---|---|---|---|---|---|
| | | | | | | | | |
| **1** | **CH**_{**2**}**Cl**_{**2**} | **2h** | **OCH**_{**3**} | **CH**_{**3**} | **C**_{**2**}**H**_{**5**} | **C**_{**2**}**H**_{**5**} | **4** | **58** |
| **2** | **-** | **-** | **-** | **-** | **CH**_{**3**} | **Bn** | **5** | **53** |
| **3** | **-** | **-** | **-** | **-** | **H** | **Bn** | **6** | **79** |
| **4** | **-** | **-** | **-** | **-** | **-** | **(CH**_{**3**}**)CHCO**_{**2**}***t* Bu** | **7** | **45** |
| | | | | | | | | |
| | | | | | | | | |
| **5** | **CH**_{**3**}**CN** | **10h** | **C**_{**2**}**H**_{**5**} | **-** | **C**_{**2**}**H**_{**5**} | **C**_{**2**}**H**_{**5**} | **8** | **88** |
| | | | | | | | | |
| **6** | **-** | **-** | **-(CH**_{**2**}**)**_{**4**} | | **-** | **-** | **9** | **92** |
| **7** | **-** | **-** | **Ph** | **H** | - | - | **10** | **82** |
| **8** | **-** | **-** | **-** | **-** | **CH**_{**3**} | **Bn** | **11** | **61** |
| **9** | **-** | **-** | **-** | **-** | **H** | **Bn** | **12** | **58** |
| **10** | **-** | **-** | **-** | **-** | **-** | **(CH**_{**3**}**)CHCO**_{**2**}***t*** | **13** | **68** |
| | | | | | | **Bu** | | |
| **11** | **-** | **8h** | ***Camphoryl*** | | **H** | **Bn** | **14** | **45** |
| **12** | **-** | **-** | ***Camphoryl*** | | **CH**_{**3**} | **Bn** | **15** | **45** |
| **13** | **-** | **-** | ***Camphoryl*** | | **C**_{**2**}**H**_{**5**} | **C**_{**2**}**H**_{**5**} | **16** | **60** |
| **14** | **-** | **-** | ***Camphoryl*** | | **-** | **(CH**_{**3**}**)CHCO**_{**2**}***t*** | **17** | **68** |
| | | | | | | **Bu** | | |

### Preparation of sulfonylesters from sulfonyl halides

### Procedure E

This procedure was developed in the case of silyl enol ethers derived from acetophenone only.

(*t*-Bu)Me₂SiOSO₂CF₃ (0.14 mmol, 0.05equiv) in anh. CH₃CN (2 mL) was degassed by freeze-thaw cycles on the vacuum line. Sulfur dioxide (1.8 mL, 41.1 mmol, 15 equiv), dried through column packed with phosphorus pentoxide and aluminum oxide, was transferred on the vacuum line to the CH₃CN solution frozen at -196°C. The mixture was allowed to melt and to warm to -78°C. After 30 min at this temperature the enoxysilane derived from acetophenone (2.74 mmol, 1 equiv) diluted in CH₃CN (2 mL) was added slowly. The mixture was stirred at -78°C 3 h. The excess of SO₂ and the solvent were evaporated under reduced pressure (10⁻³ Torr) to dryness (ca. 1h). Halogenating agent (Br₂ or NCS or NBS, 3.01 mmol, 1.1 equiv) was added at -20°C. After 1h at this temperature, the mixture was transferred into a solution of the alcohol (methanol, ethanol either in excess or 3.29 mmol, 1.2 equiv) in 2 mL CH₃CN in presence of triethylamine (3.29 mmol, 1.2 equiv) under argon atmosphere. The mixture was finally stirred at this temperature for 1 h, and poured into ice-water (20 mL) and extracted with CH₂Cl₂ (15 mL, 3 times). The combined organic extracts were washed with brine (20 mL), dried (Na₂SO₄) and the solvent eliminated under reduced pressure with reflux. The residue was purified by FC.

### Results:

### Sulfonamides

### Methyl 2-[(diethylamino) sulfonyl] propanoate (1)

Purification by FC (4:1 light petroleum ether/EtOAc, R_{f}= 0.25). Colorless oil (58%).
IR (film): ν = 2979, 1748, 1457, 1386, 1339, 1205, 1142, 1020, 943 cm⁻¹.
UV (CH₃CN): λₘₐₓ = 195 nm (ν = 5594).
¹H NMR (400 MHz, CDCl₃): δ = 3.96 (q, 1H, ³*J*= 7.1, H-C(2)), 3.79 (s, 3H, OCH₃), 3.32 (m, 4H, CH₂N), 1.61 (d, 3H, ³*J*= 7.1, Me-C(2)), 1.22 (t, 6H, ³*J*= 7.1, Me-C(2')).
¹³C NMR (100.6 MHz, CDCl₃): δ = 167.7 (s, CO), 62.4 (d, ¹*J*(C,H) = 140, C(2)), 53.0 (q, ¹*J*(C,H) = 148, OMe), 42.6 (t, ¹*J*(C,H) = 139, C(1')), 14.7 (q, ¹*J*(C,H) = 137, C(2')), 12.9 (q, ¹*J*(C,H) = 132, Me-C(2)).
CI-MS (NH₃): m/z = 241 (4, [M+18]⁺), 224 (100, [M+1]⁺).

### Methyl 2-{[benzyl (methyl) amino]sulfonyl)} propanoate (2)

Purification by FC (4:1 light petroleum ether/EtOAc, R_{f}= 0.26). Light yellow solid (53%).
IR (KBr): ν = 2953, 1744, 1455, 1438, 1339, 1201, 1152, 1136, 996, 943, 735 cm⁻¹.
UV (CH₃CN): λₘₐₓ = 211 nm (ν = 4774).
mp = 44-45°C.
¹H NMR (400 MHz, CDCl₃): δ = 7.3-7.2 (m, 5H, Ph), 4.34 (d, 1H, ²*J*= 14.4, H-C(1')), 4.25 (d, 1H, ²*J* = 14.4, H-C(1')), 4.02 (q, 1H, ³*J*= 7.1, H-C(2)), 3.74 (s, 3H, OMe), 2.73 (s, 3H, NMe), 1.59 (d, 3H, ³*J*= 7.1, Me-C(2)).
¹³C NMR (100.6 MHz, CDCl₃): δ = 167.4 (s, CO), 135.7 (s, C(Ar)), 128.6 (d, ¹*J*(C,H) = 156, C(Ar)), 128.2 (d, ¹*J*(C,H) = 159, C(Ar)), 127.9 (d, ¹*J*(C,H) = 159, C(Ar)), 61.7 (d, ¹*J*(C,H) = 140, C(2)), 52.9 (q, ¹*J*(C,H) = 148, OMe), 34.7 (q, ¹*J*(C,H) = 140, NMe), 12.8 (q, ¹*J*(C,H) = 129, Me-C(2)).
CI-MS (NH₃): m/z = 289 (27, [M+18]⁺), 272 (100, [M+1]⁺), 91 (70, [M-180]⁺).

### Methyl 2- [(benzylamino) sulfonyl] propanoate (3)

Purification by FC (4:1 light petroleum ether/EtOAc, R_{f}= 0.25). Yellow oil (79%).
IR (film): ν = 3304, 2954, 1740, 1334, 1206, 1130, 1067, 862, 700 cm⁻¹.
UV (CH₃CN): λₘₐₓ = 268 nm (ν = 4687).
¹HNMR (400 MHz, CDCl₃): δ = 7.3-7.2 (m, 5H, Ph), 5.08(br.s, 1H, H-N), 4.36 (d, 2H, ³*J*= 6.1, H-C(1')), 3.94 (q, 1H, ³*J*= 7.1, H-C(2)), 3.77 (s, 3H, OMe), 1.63 (d, 3H, ³*J*= 7.1, Me-C(2)).
¹³C NMR (100.6 MHz, CDCl₃): δ = 167.9 (s, CO), 136.8 (s, C(Ar)), 128.9 (d, ¹*J*(C,H) = 161, C(Ar)), 128.2 (d, ¹*J*(C,H) = 166, C(Ar)), 128.0 (d, ¹*J*(C,H) = 162, C(Ar)), 62.2 (d, ¹*J*(C,H) = 140, C(2)), 53.2 (q, ¹*J*(C,H) = 148, OMe), 47.9 (t, ¹*J*(C,H) = 140, C(1')), 12.7 (q, ¹*J*(C,H)= 132, Me-C(2)).
CI-MS (NH₃): m/z = 275 (16, [M+18]⁺), 258 (8, [M+1]⁺), 106 (100, [M-151]⁺).

### Tert-butyl N-[(2-methoxy-1-methyl-2-oxoethyl) sulfonyl] alaninate (4)

Purification by FC (4:1 light petroleum ether/EtOAc, R_{f}= 0.26). Light yellow oil (45%).
IR (film): ν = 3297, 2981, 2957, 1748, 1734, 1557, 1445, 1436, 1371, 1254, 1206, 1138, 1069, 982, 844 cm⁻¹ .
UV (CH₃CN): λₘₐₓ = 268 nm (ν = 2355).
*Major isomer:* ¹H NMR (400 MHz, CDCl₃): δ = 5.37 (d, 1H, ³*J*= 8.3, H-N), 4.08 (qd, 1H, ³*J* = 7.3, H-C(1')), 4.05 (q, 1H, ³*J*= 7.3, H-C(1)), 3.78 (s, 3H, OMe), 1.63 (d, 3H, ³*J*= 7.3, Me-C(1)), 1.47 (s, 9H), 1.43 (d, 3H, ³*J*= 7.3, Me-C(1')).
¹³C NMR (100.6 MHz, CDCl₃): δ = 171.8 (s, CO), 167.9 (s, COO), 82.7 (s, C-*t*Bu), 63.1 (d, ¹*J*(C,H) = 140, C(1)), 62.5 (d, ¹*J*(C,H) = 140, C(1')), 53.1 (q, ¹*J*(C,H) = 140, OMe), 27.9 (q, ¹*J*(C,H) = 148, C-*t*Bu), 20.5 (q, ¹*J*(C,H) = 130, Me-C(1')), 12.7 (q, '*J*(C,H) = 132, Me-C(1)).
*Minor isomer:* ¹H NMR (400 MHz, CDCl₃): δ = 5.27 (d, 1H, ³*J* = 8.5, H-N), 4.11 (qd, 1H, ³*J* = 7.3, H-C( 1')), 3.99 (q, 1H, ³*J* = 7.3, H-C(1)), 3.79 (s, 3H, OMe), 1.62 (d, 3H, ³*J* = 7.3, Me-C(1)), 1.47 (s, 9H), 1.42 (d, 3H, ³*J*= 7.3, Me-C(1')).
¹³C NMR (100.6 MHz, CDCl₃): δ = 171.7 (s, CO), 167.7 (s, COO), 82.6 (s, C-*t*Bu), 63.1 (d, ¹*J*(C,H) = 140, C(1)), 62.5 (d, ¹*J*(C,H) = 140, C(1')), 53.0 (q, ¹*J*(C,H) = 140, OMe), 27.9 (q, ¹*J*(C,H) = 148, C-tBu), 20.3 (q, ¹*J*(C,H) = 130, Me-C(1')), 13.0 (q, ¹*J*(C,H) = 132, Me-C(1)).
CI-MS (NH₃): m/z = 313 (100, [M+18]⁺), 296 (3, [M+1]⁺).

### N, N-diethyl-3-oxopentane-2-sulfonamide (5)

Purification by FC (4:1 light petroleum ether/EtOAc, R_{f}= 0.26). Light yellow oil (88%).
IR (film): ν = 2980, 1740, 1320, 1120, 840, 795 cm⁻¹.
UV (CH₃CN): λₘₐₓ= 197 nm (ν = 2660),216 nm (2200).
¹H NMR (400 MHz, CDCl₃): δ = 4.07 (q, 1H, ³*J* = 7.1, H-C(1')), 3.30 (q, 4H, ³*J* = 7.1, H-C(1")), 2.91 (dq, 1H, ²*J =* 14.8, ³*J* = 6.5, H-C(1)), 2.68 (dq, 1H, ²*J* = 14.8, ³*J* = 6.5, H-C(1)), 1.53 (d, 3H, ³*J* = 7.1, H-C(2')), 1.24(t, 6H, ³*J* = 7.1, Me-C(2")), 1. 11 (t, 3H, ³*J* = 6.5, Me-C(2)).
¹³C NMR (100.6 MHz, CDCl₃): δ = 203.5 (s, CO), 68.06 (d, ¹*J*(C,H) = 163.8, C(1')), 42.29 (t, ¹*J*(C,H) = 168.8, C(1")), 35.51 (t, ¹*J*(C,H) = 166.3, C(1)), 14.50 (q, ¹*J*(C,H) = 176.8, C(2")), 12.24 (q, ¹*J*(C,H) = 160.8, C(2')), 7.46 (q, ¹*J*(C,H) = 157.8, C(2)).
CI-MS (NH₃): m/z =239 (52, [M+18]⁺), 222 (70, [M+1]⁺), 72 (100, C₄H₁₀N⁺)
Anal. Calcd for C₉H₁₉NO₃S (221.31): C, 48.84; H, 8.58; Found: C, 48.98; H, 8.54.

### N,N-diethyl 2-oxo-2-phenyl ethane sulfonamide (7)

Purification by FC (4:1 light petroleum ether/EtOAc, R_{f}= 0.20). Yellow oil (82%).
IR (film): ν = 2977, 2939, 1681, 1598, 1449, 1337, 1277, 1203, 1147, 1021, 1005, 941, 757 cm⁻¹.
UV (CH₃CN): λₘₐₓ = 248 nm (ν = 6516), 208 nm (ν = 5997)
¹H NMR (400 MHz, CDCl₃): δ = 8.05 (dd, 2H, ³*J*= 8.5, ⁴*J*= 1.2, H-C(2')), 7.64 (tt, 1H, ³*J*= 7.3, ⁴*J*= 1.2, H-C(4')), 7.52 (td, 2H, ³*J*= 7.9, ⁴*J*= 1.2, H-C(3')), 4.57 (s, 2H, H-C(1)), 3.30 (q, 4H, ³*J*= 7.1, CH₂N), 1.21 (t, 6H, ³*J*= 7.1, CH₃-Et).
¹³C NMR (100.6 MHz, CDCl₃): δ = 189.2 (s, CO), 135.8 (s, C(1')), 134.2 (d, ¹*J*(C,H) = 161, C(2')), 129.2 (d, ¹*J*(C,H) = 163, C(3')), 59.1 (t, ¹*J*(C,H) = 137, C(1)), 42.9 (t, ¹*J*(C,H) = 139, C(CH₂N)), 14.7 (q, ¹*J*(C,H) = 128, C(CH₃)).
CI-MS (NH₃): m/z = 273 (5, [M+18]⁺), 256 (100, [M+1]⁺), 105 (71, [M-150]⁺).

### N-benzyl 2-oxo-2-phenyl ethane sulfonamide (8)

Purification by FC (4:1 light petroleum ether/EtOAc, R_{f}= 0.30). Yellow oil (61 %).
IR (film): ν = 3054, 2943, 1681, 1597, 1449, 1342, 1276, 1156, 994, 777 cm⁻¹.
UV (CH₃CN): λₘₐₓ = 264 nm (ν = 6805).
¹H NMR (400 MHz, CDCl₃): δ = 8.05 (dd, 2H, ³*J*= 8.0, ⁴*J*= 2.0, H-C(2')), 7.51 (tt, 1H, ³*J* = 8.0, ⁴*J*= 2.0, H-C(4')), 7.33 (td, 2H, ³*J* = 8.1, ⁴*J* = 1.8, H-C(3')), 7.26(m, 5H, H-Ph), 4.64 (s, 2H, H-C(1)), 4.34 (s, 2H, H-CH₂N), 2.81 (s, 3H, CH₃-N).
¹³C NMR (100.6 MHz, CDCl₃): δ = 187.0 (s, CO), 135.0 (d, ¹*J*(C,H) = 154, C(1')), 129.9 (m, C-Ph), 57.7 (t, ¹*J*(C,H) = 158, C(1)), 54.5 (t, ¹*J*(C,H) = 160, C(CH₂N)), 34.7 (q, ¹*J*(C,H) = 148, C(CH₃)).
CI-MS (NH₃): m/z = 320 (2, [M+18]⁺), 304 (53, [M+1]⁺), 120 (100, [M-183]⁺).

### Tert-butyl N-[(2-oxo-2-phenyl ethyl) sulfonyl] alaninate (10)

Purification by FC (4:1 light petroleum ether/EtOAc, R_{f}= 0.21). Yellow oil (68%).
IR (film): ν = 3290, 2980, 2937, 1732, 1685, 1598, 1450, 1370, 1343, 1278, 1242, 1161, 1136, 983, 753 cm⁻¹.
UV (CH₃CN): λₘₐₓ = 248 nm (ν = 7650), 208 nm (ν = 5686).
¹H NMR (400 MHz, CDCl₃): δ = 7.95 (dd, 2H, ³*J* = 8.5, ⁴*J*= 1.1, H-C(2')), 7.63 (tt, 1H, ³*J* = 7.4, ⁴*J* = 1.2, H-C(4')), 7.50 (td, 2H, ³*J* = 7.8, ⁴*J* = 1.8, H-C(3')), 5.58 (d, 1H, ³*J* = 8.5, H-N), 4.74 (d, 1H, ²*J* = 15.6, H-C(1)), 4.72 (d, 1H, ²*J* = 15.6, H-C(1)), 4.20 (qd, 1H, ³*J* = 7.1, ³*J* = 8.5, H-C(2")), 1.44 (d, 3H, ³*J* = 7.1, Me-C(2"), 1.43 (s, 9H, *t*Bu).
¹³C NMR (100.6 MHz, CDCl₃): δ = 187.7 (s, CO), 171.6 (s, COO), 135.5 (s, C(1')), 134.4 (d, ¹*J*(C,H) = 163, C(4')), 128.8 (d, ¹*J*(C,H) = 163, C(2')), 128.7 (d, ¹*J*(C,H) = 160, C(3')), 82.5 (s, C-*t*Bu), 59.6 (t, ¹*J*(C,H) = 136, C(1)), 52.9 (d, ¹*J*(C,H) = 144, C(2")), 27.8 (q, ¹*J*(C,H) = 127, C-*t*Bu), 19.7 (q, ¹*J*(C,H) = 131, Me-C(1').
CI-MS (NH₃): m/z = 345 (14, [M+18]⁺), 328 (10, [M+1]⁺), 272 (100, [M-55]⁺), 105 (94, [M-222]⁺).

### N-benzyl-1,7,7-trimethyl-2-oxo bicyclo [2.2.1] heptane-3- sulfonamide (11)

Purification by FC (4:1 light petroleum ether/EtOAc, R_{f}= 0.5). Yellow solid (45%).
IR (KBr): ν = 3445, 3308, 2965, 2941, 1742, 1332, 1321, 1155, 1041, 707, 609 cm⁻¹.
UV (CH₃CN): λₘₐₓ = 208 nm (ν = 3035), 206 nm ((ν = 3064), 199(3222), 190 ((ν = 8070).
mp = 113-114°C.
¹H NMR (400 MHz, CDCl₃): δ = 7.41-7.27 (m, 5H, Ph), 5.18 ( t, ³*J* = 6.1, HN), 4.42 (dd, 1H, ²*J* = 14.5, ³*J* = 6.4, H-C(1')), 4.33 (dd, 1H, ²*J* = 14.5, ³*J* = 6.4, H-C(1')), 3.61 (dd, 1H, ³*J* = 4.6, ⁴*J* = 1. 8, H-C(3)), 2.51 (dd, 1H, ³*J* = 4.3, ³*J* = 4.3 H-C(4)), 2.30 (td, 1H, ²*J* = 13 .0, ³*J* = 8.6, H-C(5)), 1.85 (m, 1H, H-C(5)), 1.71 (ddd, 2H, ²*J* = 14.4, ³*J* = 9.0, H-C(6)), 1.00 (s, 3H, Me-C(7)), 0.93 (s, 3H, Me-C(7)), 0.66 (s, 3H, Me-C(1)).
¹³C NMR (100.6 MHz, CDCl₃): δ = 208.5 (s, CO), 136.2 (s, C(Ar)), 129.0 (d, ¹*J*(C,H)= 153,C(Ar)), 128.3 (d, ¹*J*(C,H) = 160,C(Ar)), 128.2 (d, ¹*J*(C,H) = 160, C(Ar)), 69.7 (d, ¹*J*(C,H) = 135, C(3)), 59.4 (s, C(1)), 47.6 (d, ¹*J*(C,H) = 148, C(4)), 46.6 (t, ¹*J*(C,H)= 140, C(1')), 45.9 (s, C(7)), 30.2 (t, ¹*J*(C,H) = 134, C(6)), 21.6 (t, ¹*J*(C,H) = 137, C(5)), 19.5 (t, ¹*J*(C,H) = 125, Me-C(7)), 18.5 (q, ¹*J*(C,H) = 126, Me-C(7)), 9.6 (q, ¹*J*(C,H) = 127, Me-C(4)).
EI-MS: m/z = 321 (2, [M+1]⁺), 106 (100, [M-215]).

### N-benzyl-N,1,7,7-tetramethyl-2-oxo bicyclo [2.2.1] heptane-3- sulfonamide (12)

Purification by FC (9:1 light petroleum ether/EtOAc, R_{f}= 0.3). Colorless solid (45%).
IR (KBr): ν = 2970, 2929, 1745, 1333, 1149, 992, 938, 767, 736 cm⁻¹.
UV (CH₃CN): λₘₐₓ = 211 nm (ν = 5395).
mp = 92-93°C.
¹H NMR (400 MHz, CDCl₃): δ = 7.41-7.27 (m, 5H, Ph), 4.61(d, 1H, ²*J*= 14.7, H-C(1')), 4.31 (d, 1H, ²*J* = 14.7, H-C(1')), 3.82 (dd, 1H, ³*J* = 4.4, ⁴*J* = 1.8, H-C(3)), 2.87 (s, 3H, MeN ) 2.55 (dd, 1H, ³*J* = 4.1, ³*J* = 4.1 H-C(4)), 2.36 (td, 1H, ²*J* = 13.2, ³*J* = 7.1, H-C(5)) 1.93 (m, 1H, H-C(5)), 1.76 (ddd, 2H, ²*J* = 14.7, ³*J* = 8.2, H-C(6)), 1.05 (s, 3H, Me-C(7)), 0.96 (s, 3H, Me-C(7)), 0.85 (s, 3H, Me-C(1)).
¹³C NMR (100.6 MHz, CDCl₃): δ = 208.5 (s, CO), 136.2 (s, C(Ar)), 128.8 (d, ¹*J*(C,H) = 153,C(Ar)), 128.4 (d, ¹*J*(C,H) = 160,C(Ar)), 127.9 (d, ¹*J*(C,H) = 160,C(Ar)), 69.3. (d, ¹*J*(C,H) = 135, C(3)), 59.4 (s, C(1)), 54.3 (d, ¹*J*(C,H) = 140, C(1')), 47.3 (d, ¹*J*(C,H) = 148, C(4)), 45.9 (s, C(7)), 34.6 (q, ¹*J*(C,H) = 140, MeN), 29.7 (t, ¹*J*(C,H) =134, C(6)), 22.0 (t, ¹*J*(C,H) = 137, C(5)), 19.7 (t, ¹*J*(C,H) = 125, Me-C(7)), 18.7 (q, ¹*J*(C,H) = 126, Me-C(7)), 9.8 (q, ¹*J*(C,H) = 127, Me-C(4)).
CI-MS (NH₃): m/z = 353 (59, [M+18]⁺), 336 (53, [M+1]⁺), 170 (100, [M-165]⁺, 153 (52, [M-182]⁺)

### N,N-diethyl-1,7,7-trimethyl-2-oxo bicyclo [2.2.1] heptane-3- sulfonamide (13)

Purification by FC (4:1 light petroleum ether/EtOAc, R_{f}= 0.5). Colorless solid(60%).
IR (KBr): ν = 2967, 1741, 1446, 1324, 1137, 1015, 939, 684 cm⁻¹.
UV (CH₃CN): λₘₐₓ = 194 nm (ν = 3036).
mp = 70-71 °C.
¹H NMR (400 MHz, CDCl₃): δ = 3.77 (dd, 1H, ³*J* = 4.6, ⁴*J* = 1.8, H-C(3)), 3.48(dq, 1H, ²*J* = 14.5, ³*J* = 7.1, CH₂N), 4.27 (dq, 1H, ²*J* = 14.5, ³*J* = 7.1, CH₂N), 2.51 (dd, 1H, ²*J* = 4.3, ²*J* = 4.3, H-C(4)), 2.31 (td, 1H, ²*J* = 13.2, ³*J* = 7.4, H-C(5)), 1.86 (m, 1H, H-C(5)), 1.71 (ddd, 2H, ²*J* = 14.4, ³*J* = 7.9, H-C(6)), 1.21 (t, 3H, ³*J* = 7.1, CH₃- Et), 1.04 (s, 3H, Me-C(7)), 0.95 (s, 3H, Me-C(7)), 0.87 (s, 3H, Me-C(1)).
¹³C NMR (100.6 MHz, CDCl₃): δ = 208.5 (s, CO), 70.5 (d, ¹*J*(C,H) = 136, C(3)), 59.3 (s, C(1)), 47.5 (d, ¹*J*(C,H) = 148, C(4)), 45.6 (s, C(7)), 42.1 (t, ¹*J*(C,H) = 139, C(CH₂N)), 29.7 (t, ¹*J*(C,H) = 134, C(6)), 21.8 (t, ¹*J*(C,H) = 137, C(5)), 19.7 (q, ¹*J*(C,H) = 125, Me-C(7)), 18.6 (q, ¹*J*(C,H) = 126, Me-C(7)), 14.8 (q, ¹*J*(C,H) = 127, C(CH₃), 9.7 (q, ¹*J*(C,H) = 127, Me-C(4)).
CI-MS (NH₃): m/z = 305 (16, [M+18]⁺), 288 (100, [M+1]⁺), 124 (17, [M-163]⁺).

### Tert-butyl N-[(1,7,7-trimethyl-2-oxobicyclo [2.2.1] hept-3-yl) sulfonyl] alaninate (14)

Purification by FC (4:1 light petroleum ether/EtOAc, R_{f}= 0.48). Light yellow oil (68%).
IR (film): ν = 3290, 2969, 2936, 1744, 1340, 1159, 1135, 734, 603 cm⁻¹.
UV (CH₃CN): λₘₐₓ = 196 nm (ν = 4425).
¹H NMR (400 MHz, CDCl₃): δ = 5.44 (d, 1H, ³*J* = 8.8, HN), 4.1 (m, 2H, H-C(3), H-C(1')), 2.56 (t, 1H, ³*J* = 4.1, H-C(4)), 2.25 (ddd, 1H, ²*J* = 14.8, ³*J* = 8.2, ³*J* = 4.4; H-C(1)), 1.85 (m, 1H, H-C(5)), 1.71 (m, 2H, H-C(6)), 1.46 (d, 3H, ³*J* = 7.1, Me-C(1')), 1.45 (s, 9H, *t*Bu), 1.0 (s, 3H, Me-C(7)), 0.96 (s, 3H, Me-C(4)).
¹³ C NMR (100.6 MHz, CDCl₃): δ = 208.8 (s, CO), 171.9 (s, COO), 82.6 (s, C(*t*Bu)), 70.7 (d, ¹*J*(C,H) = 135, C(3)), 59.4 (s, C(1)), 52.9 (d, ¹*J*(C,H) = 144, C(1')), 46.8 (d, ¹*J*(C,H) = 149, C(4)), 45.9 (s, C(7)), 30.3 (t, ¹*J*(C,H) = 134, C(6)), 27.9 (q, ¹*J*(C,H) = 127, Me-*t*Bu), 21.5 (t, ¹*J*(C,H) = 137, C(5)), 20.1 (t, ¹*J*(C,H) = 135, Me-C(1')), 19.7 (q, ¹*J*(C,H) = 125, Me-C(7)), 9.7 (q, ¹*J*(C,H) = 128, Me-C(4)).
CI-MS (NH₃): m/z = 377 (14, [M+18]⁺), 360 (5, [M+1]⁺), 304 (100, [M-55]⁺), 258 (55, [M-101]⁺).

### Sulfonylesters

### Isopropyl 2-oxo-2-phenyl ethane sulfonate(23)

by FC (9:1 light petroleum ether/EtOAc, R_{f}= 0.18). Light yellow solid (30%).
IR (KBr): ν = 2987, 1686, 1598, 1450, 1360, 1278, 1175, 1094, 916 cm⁻¹.
¹HNMR (400 MHz, CDCl₃): δ = 8.01 (dd, 2H, ³*J* = 8.2, ⁴*J* = 1.3, H-C(2')), 7.67 (tt, 1H, ³*J* = 7.4, ⁴*J* = 1.3, H-C(4')), 7.53 (td, 2H, ³*J* = 8.2, ⁴*J* = 1.3, H-C(3')), 5.05(q, 1H, ³*J* = 6.08, H-C(1")), 4.69 (s, 2H, H-C(1)), 1.42 (d, 3H, ³*J*= 6.08, H-C(2")).
¹³C NMR (100.6 MHz, CDCl₃): δ = 187.7 (s, CO), 135.7 (s, C(1')), 134.9 (d, ¹*J*(C,H) = 162, C(4')), 129.2 (d, ¹*J*(C,H) = 163, C(2')), 129.0 (d, ¹*J*(C,H) = 162, C(3')), 79.6 (d, ¹*J*(C,H) = 151, C(1")), 58.2 (t, ¹*J*(C,H) = 138, C(1)), 23.1 (q, ¹*J*(C,H) = 128, C(2"))
CI-MS (NH₃): m/z = 260 (1, [M+18]⁺), 243 (1, [M+1]⁺), 105 (100, [M-137]⁺).

### Phenyl 2-oxo-2-phenylethane sulfonate(24)

Purification by FC (9:1 light petroleum ether/EtOAc, R_{f}= 0.19). Colourless soild (35%).
IR (KBr): ν = 3065, 2972, 1686, 1597, 1587, 1488, 1450, 1380, 1278, 1192, 1143, 868, 784, 688 cm⁻¹.
UV (CH₃CN): λₘₐₓ = 249nm (ν = 9369), 211 nm (ν = 7797).
¹H NMR (400 MHz, CDCl₃): δ = 8.03 (dd, 2H, ³*J* = 8.2, ⁴*J* = 1.3, H-C(2')), 7.67 (tt, 1H, ³*J* = 7.4, ⁴*J* = 1.3, H-C(4')), 7.54 (td, 2H, ³*J* = 8.2, ⁴*J* = 1.3, H-C(3')), 7.4 (m, 5H, Ph), 4.81 (s, **2H**, H-C(1)).
¹³C NMR (100.6 MHz, CDCl₃): δ = 186.6 (s, CO), 149.4 (s, C(Ar)), 135.4 (s, C(1')), 134.8 (d, ¹*J*(C,H) = 163, C(4')), 132.8 (d, ¹*J*(C,H) = 163, C(Ar)), 130.2 (d, ¹*J*(C,H) = 165, C(Ar)), 129.4 (d, ¹*J*(C,H) = 164, C(2')), 129.1 (d, ¹*J*(C,H) = 163; C(3')), 127.8 (d, ¹*J*(C,H) = 168, C(Ar)), 56.3(t, ¹*J*(C,H) = 139, C(1)).
CI-MS (NH₃): m/z = 294 (6, [M+18]⁺), 277 (2, [M+1]⁺), 121 (88, [M-156]⁺), 105 (100, [M-172]⁺).

## Claims

1. A process for preparing sulfonyl halides comprising the following steps:
a) reacting an enoxymetal, allylsilane or allylstannane compound with sulphur dioxide in the presence of a suitable Lewis acid, in a suitable solvent, at a suitable temperature, and for a time sufficient to produce a metal sulfinate,
b) adding a suitable halogen source to the metal sulfinate of step a) and reacting said components in a suitable solvent, at a suitable temperature, and for a time sufficient to produce a sulfonyl halide.

2. The process according to claim 1, **characterized in that** in step a) an enoxymetal, allylsilane or allylstannane compound and a compound of the general formula A: 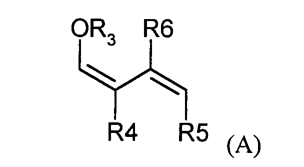 wherein
R₃ denotes C₁₋₁₀ alkyl, aryl, arylalkyl, or hetarylalkyl, preferably C₁₋₆ alkyl, benzyl, arylmethyl, or hetarylmethyl, more preferably C₁₋₄ alkyl, H, benzyl, or furfuryl, with the proviso that any R₃ containing a free amine base or a thiol or thiolether cannot be used.
R₄ denotes H or C₁₋₁₀ alkyl, preferably H or C₁₋₆ alkyl, more preferably H or C₁₋₄ alkyl,
R₅ denotes H or C₁₋₁₀ alkyl, preferably H or C₁₋₆ alkyl more preferably H or C₁₋₄ alkyl,
with the proviso that any R₄, R₅ containing a free amine, amide, thioether cannot be used.
R₆ denotes H or acyloxy, preferably H, methylcarboxy, ethylcarboxy, i-propylcarboxy, t-butylcarboxy, or arylcarboxy, more preferably phenylcarboxy
are reacted with sulphur dioxide in the presence of a suitable Lewis acid, in a suitable solvent, at a suitable temperature, and for a time sufficient to produce a metal sulfinate.

3. The process according to claim 1 or 2, **characterized in that** the metal of the enoxymetal compound is selected from Si and Sn, preferably Si.

4. The process according to anyone of claims 1 to 3, **characterized in that** the source of halogen is elemental halogen or a halo-succinimide compound, preferably chlorine, bromine, N-chlorosuccinimide, N-iodosuccinimide, or N-bromosuccinimide, more preferably N-chlorosuccinimide, N-iodosuccinimide, or N-bromosuccinimide, most preferably N-chlorosuccinimide.

5. The process according to any one of claims 1 to 4, **characterized in that** the temperature in step b) is 40 to -50 °C, preferably 10 to -40 °C, more preferably -10 to -30 °C, most preferably about -20 °C.

6. The process according to any one of claims 1 to 5, **characterized in that** the solvent in step b) is selected from the group consisting of methylene dichloride, acetonitrile, or anhydrous tetrahydrofurane, preferably the solvent is methylene dichloride, and most preferably no solvent except for the source of halogen is used.

7. The process according to any one of claims 1 to 6, **characterized in that** the reaction time in step b) is from 15 minutes to 20 hours, preferably from 30 minutes to 15 hours, more preferably from 1 hour to 8 hours, most preferably from 2 to 4 hours.

8. The process according to any one of claims 1 to 7, **characterized in that** in step b) no solvent is used, and the suitable temperature is -10 to -30 °C, preferably about -20 °C, and the reaction time is from 1 to 12 hours.

9. The process according to any one of claims 1 to 8, **characterized in that** the product of step b) is the sulfonyl halide of
a) general formula I: 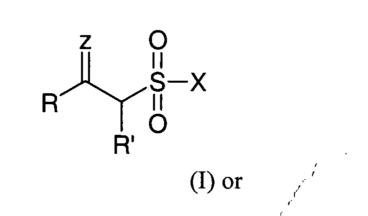
b) general formula II: 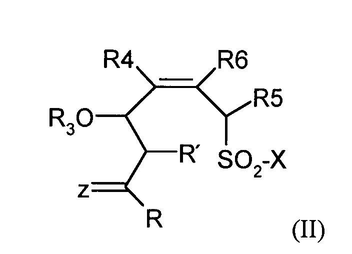
wherein
Z denotes CH₂ or O, preferably O
R, R', denote, in each case independently of one another, hydrogen, C₁₋₁₀ alkyl, C₁₋₁₀ alkoxy, aryl, hetaryl, preferably pyridine, pyrazine, pyrimidine, arylalkyl, hetarylalkyl, phenyl, or R and R" denote together a (CH₂)₃₋₆ ring or a camphoryl that may substituted by aryl, acylalkyl, hetaryl, hetarylalkyl, C₁₋₁₀ alkyl, and/or C₁₋₁₀ alkoxy, and
R₃ denotes C₁₋₁₀ alkyl, aryl, arylalkyl, hetaryl, or hetarylalkyl, preferably C₁₋₆ alkyl, benzyl, arylmethyl, or hetarylmethyl, more preferably C₁₋₄ alkyl, H, benzyl or furfuryl, with the proviso that any R₃ containing a free amine base or a thiol or a thiolether cannot be used.
R₄ denotes H or C₁₋₁₀ alkyl, preferably H or C₁₋₆ alkyl, more preferably H or C₁₋₄ alkyl.
R₅ denotes H or C₁₋₁₀ alkyl, preferably H or C₁₋₆ alkyl more preferably H or C₁₋₄ alkyl,
with the proviso that any R₄, R₅ containing a free amine, amide, thioether cannot be used.
R₆ denotes H or acyloxy, preferably H, methylcarboxy, ethylcarboxy, i-propylcarboxy, t-butylcarboxy, or arylcarboxy, more preferably phenylcarboxy
X denotes halogen, preferably chlorine, bromine or iodine.

10. The process according to any one of claims 1 to 9, **characterized in that** step b) is followed by a further step c), said step c) comprising the addition of an amine to the product of step b) and reacting said components in a suitable basic solvent, at a suitable temperature, and for a reaction time sufficient to produce a sulphonamide.

11. The process according to claim 10, **characterized in that** the amine is a protected amino acid, more preferably an amino acid t-butylester, or the amine that has the general formula NR₁R₂ (III), wherein R₁, R₂ denote, in each case independently of one another, hydrogen, C₁₋₁₀ alkyl, aryl, arylalkyl, hetaryl, hetarylalkyl, or R₁ and R₂ form a cyclic system, preferably a saturated 3-10 or unsaturated 5-10 membered ring, more preferably benzyl.

12. The process according to claims 10 or 11, **characterized in that** the suitable solvent is pyridine or dichloromethane and a suitable base, preferably triethylamine or diethylisopropylamine.

13. The process according to anyone of claims 10 to 12, **characterized in that** the suitable temperature is 30 to -50 °C, preferably 10 to -40 °C, more preferably -10 to -30 °C, most preferably about -20 °C.

14. The process according to anyone of claims 10 to 13, **characterized in that** the suitable reaction time is 30 minutes to 6 hours, preferably 1 hour to 4 hours, more preferably about 2 hours.

15. The process according to anyone of claims 1 to 9, **characterized in that** step b) is followed by a further step c), said step c) comprising the addition of an alcohol to the product of step b) and reacting said components in a suitable basic solvent, at a suitable temperature, and for a reaction time sufficient to produce a sulphonate.

16. The process according to claims 15, **characterized in that** the suitable solvent is dichloromethane in combination with a suitable base, preferably triethylamine, NaOH or a suitable carbonate.

17. The process according to any one of claims 15 or 16, **characterized in that** the suitable temperature is 0 to -50 °C, preferably -10 to -40 °C, more preferably -10 to -30 °C, most preferably about -20 °C.

18. The process according to anyone of claims 15 to 17, **characterized in that** the suitable reaction time is 30 minutes to 6 hours, preferably 1 hour to 4 hours, more preferably about 2 hours.

19. The process according to anyone of claim 15 to 18, **characterized in that** the alcohol alcohol is a C₁₋₁₀ alcohol, arylalcohol, arylmethanol, hetarylmethanol, or phenol, preferably a C₁₋₆ alcohol or phenol or benzyl alcohol, more preferably a C₁₋₄ alcohol.

20. The process according to anyone of claims 1 to 19, **characterized that** all steps are performed in one reaction vessel consecutively "one pot reaction".

21. A sulfonyl compound of the
a) general formula IV: 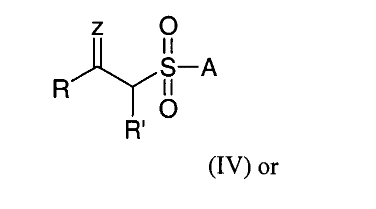
b) general formula V: 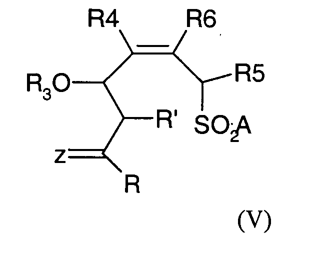
wherein
Z denotes CH₂, O, preferably O
R, R', denote, in each case independently of one another, hydrogen, C₁₋₁₀ alkyl, C₁₋₁₀ alkoxy, aryl, hetaryl, preferably pyridine, pyrazine, pyrimidine, arylalkyl, hetarylalkyl, phenyl, or R and R" denote together a (CH₂)₃₋₆ ring or a camphoryl that may substituted by aryl, acylalkyl, hetaryl, hetarylalkyl, C₁₋₁₀ alkyl, and/or C₁₋₁₀ alkoxy, and
R₃ denotes C₁₋₁₀ alkyl, aryl, arylalkyl, hetaryl, or hetarylalkyl, preferably C₁₋₆ alkyl, benzyl, arylmethyl, or hetarylmethyl, more preferably C₁₋₄ alkyl, H, benzyl or furfuryl, with the proviso that any R₃ containing a free amine base or a thiol or a thiolether cannot be used.
R₄ denotes H or C₁₋₁₀ alkyl, preferably H or C₁₋₆ alkyl, more preferably H or C₁₋₄ alkyl.
R₅ denotes H or C₁₋₁₀ alkyl, preferably H or C₁₋₆ alkyl more preferably H or C₁₋₄ alkyl,
with the proviso that any R₄, R₅ containing a free amine, amide, thioether cannot be used.
R₆ denotes H or acyloxy, preferably H, methylcarboxy, ethylcarboxy, i-propylcarboxy, t-butylcarboxy, or arylcarboxy, more preferably phenylcarboxy
A denotes
i) halogen, preferably chlorine, iodine or bromine, or
ii) a C₁₋₁₀ alcohol, arylalcohol, arylmethanol, hetarylmethanol, or phenol, preferably a C₁₋₆ alcohol or phenol or benzyl alcohol, more preferably a C₁₋₄ alcohol,
iii) an amino acid or an amine that has the general formula NR₁,R₂ (III), wherein R₁, R₂ denote, in each case independently of one another, hydrogen, C₁₋₁₀ alkyl, aryl, arylalkyl, hetaryl, hetarylalkyl, or R₁ and R₂ form a cyclic system, preferably a saturated 3-10 or unsaturated 5-10 membered ring, more preferably benzyl,
iv) a heterocyclic system selected from the group consisting of: 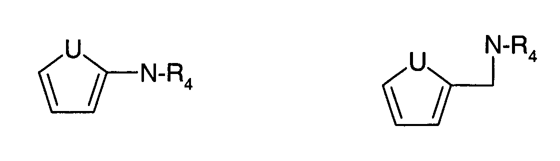 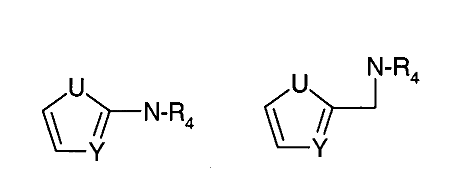 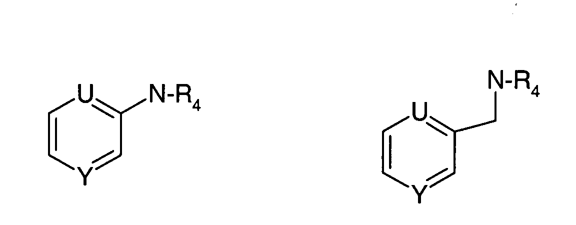
wherein
U denotes O, N-R₄, S, CH, N
Y denotes N, CH
with the proviso that in a heteroyclic ring containing U and Y, U denotes N and Y denotes N or CH.

22. The compound according to claim 21, **characterized in that** it has the general formula VI: 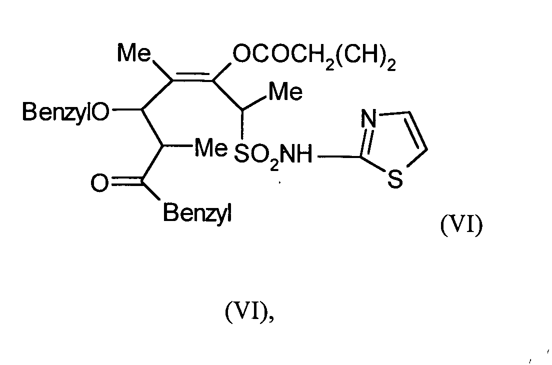 preferably: (1Z, 3R,4R)-3-(benzyloxy)-2,4 dimethyl -5-oxo-1-{1-[((1,3-thiazol-2-ylamino)sulfonyl)ethyl}hex-1-enyl carbonate.

23. The compound according to claim 21 or 22, **characterized in that** A denotes an alcohol.

24. Compounds according to claim 23 selected from the group consisting of:
Methyl 2-oxo-2-phenylethane sulfonate,
Ethyl 2-oxo-2-phenylethane sulfonate
Phenyl 2-oxo-2-phenylethane sulfonate, and
Isopropyl 2-oxo-2-phenyl ethane sulfonate.

25. Compounds according to anyone of claims 23 or 24 for the preparation of a medicament, a bacteriocide, a pesticide, a herbicide, or a fungicide.

26. Use of a compound according to anyone of claims 23 or 24 for the preparation of a medicament for the treatment of bacterial or fungal infections.

27. Pharmaceutical composition comprising a compound according to anyone of claims 22 or 23.

28. The compound according to anyone of claims 21 or 22, **characterized in that** A denotes an amine.

29. Compounds according to claim 28 selected from the group consisting of:
Tert-butyl N-[(1,7,7-trimethyl-2-oxobicyclo [2.2.1] hept-3-yl) sulfonyl] alaninate,
N,N-diethyl-1,7,7-trimethyl-2-oxo bicyclo [2.2.1] heptane-3- sulfonamide,
N-benzyl-N,1,7,7-tetramethyl-2-oxo bicyclo [2.2.1] heptane-3- sulfonamide,
N-benzyl-1,7,7-trimethyl-2-oxo bicyclo [2.2.1] heptane-3- sulfonamide,
Tert-butyl N-[(2-oxo-2-phenyl ethyl) sulfonyl] alaninate,
N-benzyl 2-oxo-2-phenyl ethane sulfonamide,
N,N-diethyl 2-oxo-2-phenyl ethane sulfonamide,
N, N-diethyl-3-oxopentane-2-sulfonamide,
Tert-butyl N-[(2-methoxy-1-methyl-2-oxoethyl) sulfonyl] alaninate,
Methyl 2- [(benzylamino) sulfonyl] propanoate,
Methyl 2-{[benzyl (methyl) amino]sulfonyl)} propanoate, and
Methyl 2-[(diethylamino) sulfonyl] propanoate

30. Compounds according to claim 28 or 29 for the preparation of a medicament.

31. Use of a compound according to anyone of claims 28 to 29 for the preparation of a medicament for the treatment of bacterial or fungal infections.

32. Pharmaceutical composition comprising a compound according to anyone of claims 28 or 29.

33. Use of a compound according to claim 21, wherein A denotes a halogen for the preparation of a sulphonamide or a sulfonate.
